# EUROPEAN PATENT APPLICATION

(11) **EP 4 775 231 A1**
(43) Date of publication of application: **15.07.2026**
(21) Application number: 24863208.5
(22) Date of filing: 05.09.2024
(51) Int. Cl.: A61L 27/20, A61L 27/52, A61L 27/12

(54) **COMPOSITION FOR PREPARING BIOMATERIAL FOR IN VIVO ION-SENSITIVE TISSUE REPAIR AND USE THEREOF**

(30) Priority: 06.09.2023 KR 20230118550
(71) Applicant: Medifab Co., Ltd., Seoul 08594 (KR)
(72) Inventor: KIM, Su Hee, Seoul 08594 (KR); CHA, Mi Sun, Seoul 08594 (KR); LEE, Soo Hee, Seoul 08594 (KR); KANG, Jae Jun, Seoul 08594 (KR)
(74) Representative: Morabito, Sara
(86) International application number: PCT/KR2024/013383
(87) International publication number: WO 2025/053629

(57) **Abstract**

The present application relates to a composition for producing a biomaterial for *in vivo* ion-sensitive tissue repair and use thereof, and provides a composition for preparing an *in vivo* ion-sensitive hydrogel composition including a solution containing a chitosan derivative in which a degree of deacetylation, a degree of substitution of alkylcarboxyl groups, and a level of free amine groups are each controlled, or a solution containing the chitosan derivative, and an *in vivo* ion-sensitive hydrogel composition using the composition.

## Description

### Technical Field

The present disclosure relates to a composition for preparing a biomaterial for *in vivo* ion-sensitive tissue repair, and use thereof. This application claims priority to Korean Patent Application No. 10-2023-0118550, filed on September 6, 2024, and the disclosure of the patent application is incorporated herein by reference.

### Background Art

Aging of the skin is a gradual phenomenon that occurs over time and is influenced by lifestyle factors such as alcohol consumption, smoking, and exposure to ultraviolet rays. Among these, aging of facial skin is characterized by atrophy, sagging, and hypertrophy. Atrophy refers to a significant decrease in the thickness of skin tissue, sagging of the subcutaneous tissue causes excess skin and ptosis, and leads to drooping of the cheeks and eyelids. Additionally, hypertrophy refers to excessive weight gain due to swelling of the face and neck area. These changes are typically associated with loss of elasticity and rough skin surface condition.

A hydrogel is a material having a polymer network structure containing a large amount of moisture, and refers to a material formed of a homopolymer, a copolymer, or the like. Hydrogels swell and become transparent through the absorption of hydrophilic components including water in an aqueous solution, and have appropriate mechanical properties. Such a degree of swelling and mechanical properties of a polymer depends mainly on the physical properties of the polymer network structure and the formulation method. As materials capable of preparing such hydrogels, polymers are mainly used, and the polymers are classified into natural polymers and synthetic polymers according to the origin of the materials. Hydrogels made from natural polymers may give consumers a good impression of being naturally derived because they are made from polymers derived from natural materials such as agar, chitosan, carrageenan, cellulose, and xanthan gum, but they are characterized by weak mechanical properties. Since most of these natural polymers are made by physical gelation reactions, the hydrogel shape easily collapses in high-temperature conditions, and the mechanical strength weakens even in low-temperature conditions. Moreover, even in room-temperature conditions, the appearance of the hydrogel may be easily broken or torn by a weak external force such as pressing by a finger, and therefore substantial caution is required during use and distribution. In contrast, hydrogels made from synthetic polymers have strong bonding forces, such as chemical covalent bonds between molecules through cross-linking, and therefore, unlike natural polymers, they have strong mechanical properties, and therefore it is relatively rare for a hydrogel form to be damaged by external stimuli such as temperature and external forces. However, in the case of synthetic polymer-based hydrogels, improvement is required in terms of biocompatibility and maintenance of efficacy.

In particular, as injectable liquid preparations, existing biomaterials for tissue repair have characteristics of dissolving under acidic conditions, which causes pain when injected into the skin, and under neutral conditions, the stability of the material when mixed with another material or component decreases, and in some cases, precipitates form within the material. Against this technical background, there is a need to develop next-generation biomaterials for tissue repair that may maximize the advantages of formulations using existing natural/synthetic polymers, while compensating for existing disadvantages and achieving the ultimate objective of replacement with autologous tissue (Korean Registered Patent No. 10-0506543).

### Disclosure of Invention

### Technical Problem

One aspect is to provide a composition for preparing an *in vivo* ion-sensitive hydrogel composition including a solution including a chitosan derivative in which a degree of deacetylation, a degree of substitution of alkyl carboxyl groups are each controlled, and a level of free amine groups.

Another aspect is to provide a method of preparing an *in vivo* ion-sensitive hydrogel composition, the method including mixing a solution containing a chitosan derivative in which a degree of deacetylation, a degree of substitution of alkyl carboxyl groups, and a level of free amine groups are each controlled, and an aqueous solution containing phosphate ions.

Another aspect is to provide an *in vivo* ion-sensitive hydrogel composition prepared by the method.

Another aspect is to provide a treatment method using the *in vivo* ion-sensitive hydrogel or tissue repair biomaterial.

### Solution to Problem

One aspect is to provide a composition for preparing an *in vivo* ion-sensitive hydrogel composition including a solution containing a chitosan derivative satisfying the following conditions:
(a) Having a degree of deacetylation of 70 % or more;
(b) 50 % or more of the hydroxyl groups and amine groups in the chitosan derivative are substituted with O-alkylcarboxyl groups or N-alkylcarboxyl groups; and
(c) 22 % or more of the total amine groups in the chitosan derivative shall be free amine groups (-NH₂).

As used herein, the term "hydrogel" may refer to a three-dimensional network structure formed by cross-linking a hydrophilic polymer through covalent or noncovalent bonds. Due to the hydrophilicity of constituent materials, a hydrogel absorbs a large amount of water in an aqueous solution and under an aqueous environment, and swells but has the property of not dissolving due to the cross-linked structure. Therefore, various forms and properties of hydrogels may be prepared depending on constituent components and a manufacturing method, and since in general, a hydrogel contains a large amount of moisture and may have intermediate properties between a liquid and a solid.

As an example, the term "hydrogel" may be used interchangeably with the term "biomaterial for tissue repair" or "biomaterial composition for tissue repair." In an embodiment, the hydrogel may be a biomaterial for tissue repair, which may be a material used for tissue repair. For example, it may refer to a filler material similar to soft tissue injected into skin having wrinkles or into a region requiring volume, an adhesion-preventing material between a surgical site and normal tissue, a tissue adhesive material, or a wound covering material for artificial skin, etc. The hydrogel may be applied to, for example, glabella, forehead, under-eye aegyo-sal, crow's feet, nasolabial folds, cheek, perioral wrinkles, and chin, etc. As a material applied directly to the human body, the hydrogel must have biocompatibility. For example, when a hydrogel is filled into a region requiring volume, excellent retention/persistence of the gel shape is required to form volume for a long period after injection. When the hydrogel is used for adhesion prevention at a surgical site, it must have tissue compatibility at a wound site and have low or no cytotoxicity.

As used herein, the term "for tissue repair" refers to restoring a structure and a function of damaged or aged tissue, and may include, for example, a cosmetic filler use, an adhesion-preventing material use, an adhesive use, a wound covering material use, and an implant use for molding, etc. but is not limited thereto.

As used herein, the term "ion-sensitive" refers to a physical property in which a formulation changes depending on a surrounding ionic environment, and may refer to a characteristic in which, under room temperature conditions, for example, under *in vitro* conditions and an environment exposed to room temperature, the formulation exists in a liquid formulation, that is, in a sol form, and under *in vivo* conditions, is converted into a gel form.

As used herein, the term "chitosan" may refer to a linear polysaccharide composed of D-glucosamine and N-acetylglucosamine. The chitosan may include a material obtained by deacetylating chitin using an alkali, etc. More specifically, the chitosan refers to a polymer material having a ratio of D-glucosamine generated by removal of an acetyl group from N-acetyl-D-glucosamine is 70 % or more, and may be represented by the following Structural Formula 1.

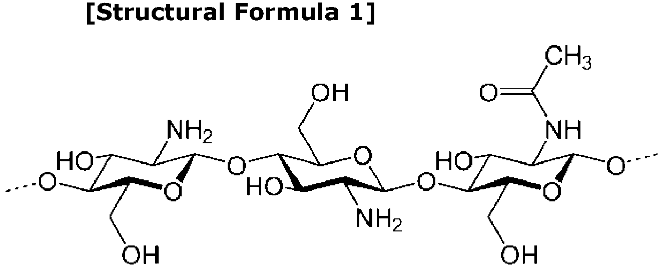

As used herein, the term "chitosan derivative" may refer to a material derived from the aforementioned chitosan, and a structure in which an organic chemical modification including introduction of a specific functional group, oxidation, reduction, substitution of atoms, etc. has been introduced. In the related art, existing chitosan derivatives act as weak polyanionic polyelectrolytes, and therefore, under neutral conditions, amino groups in the chitosan derivative are not protonated and most carboxyl groups are not dissolved, and thus there is a limitation in using them as biomaterials. Accordingly, there is a need for development of a new technology for improving applicability of a chitosan derivative as a biomaterial. The chitosan derivative may be, for example, a carboxyalkyl chitosan, and specifically, may be composed of a combination of a plurality of monomer units represented by the following Structural Formula 2.

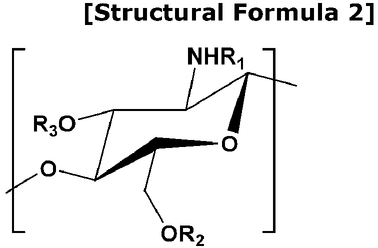

In the Structural Formula 2, R₁ may be any one of hydrogen, an alkylcarboxyl group, and an acetyl group, and R₂ and R₃ may independently be hydrogen or an alkylcarboxyl group. Here, the alkylcarboxyl group may be, for example, a methylcarboxyl group or an ethylcarboxyl group, and specifically may be a methylcarboxyl group.

In an embodiment, an average molecular weight of the chitosan derivative may be 50,000 to 3,000,000 Da. The average molecular weight originates from a plurality of monomer units represented by the aforementioned Structural Formula 1 or Structural Formula 2, or a combination thereof, and may be appropriately modified within an ordinary range known in the art.

In an embodiment, the chitosan derivative may exhibit high solubility and stability under neutral conditions, while simultaneously having the characteristics of being able to gel under *in vivo* conditions to form a matrix with various viscoelasticities. To this end, the chitosan derivative may have a degree of deacetylation of 70 % or more, a degree of substitution of an O-alkylcarboxyl group or an N-alkylcarboxyl group of 50 % or more, and a level of free amine groups of 22 % or more. In addition, a pH of a solution or *in vivo* ion-sensitive hydrogel composition containing the chitosan derivative according to an embodiment may be pH 6.5 to pH 7.5, for example, may be pH 6.5 to pH 7.5, pH 6.8 to pH 7.5, pH 7.1 to pH 7.5, pH 7.4 to pH 7.5, pH 6.5 to pH 7.2, pH 6.8 to pH 7.2, pH 7.1 to pH 7.2, pH 6.5 to pH 6.9, pH 6.8 to pH 6.9, or pH 6.5 to pH 6.6. The degree of deacetylation, the degree of substitution of an O-alkylcarboxyl group or an N-alkylcarboxyl group, and the level of free amine groups of the aforementioned chitosan derivative are parameters that may be controlled according to techniques known in the art and may include, for example, a series of processes of reacting chitosan powder having a specific degree of deacetylation with an appropriate amount of monochloroacetic acid, but are not limited thereto.

For example, the degree of deacetylation, the degree of substitution of an O-alkylcarboxyl group or an N-alkylcarboxyl group, etc. may be controlled according to the following method: To obtain a chitosan derivative having different levels of deacetylation, 10 g of chitin may be reacted in 100 mL of a 50 % NaOH solution at 100 °C for 30 minutes to 150 minutes, for example, 30 minutes to 120 minutes, 30 minutes to 90 minutes, 30 minutes to 60 minutes, 60 minutes to 150 minutes, 60 minutes to 140 minutes, 60 minutes to 130 minutes, 60 minutes to 120 minutes, 60 minutes to 110 minutes, 60 minutes to 100 minutes, 60 minutes to 90 minutes, 60 minutes to 80 minutes, or 60 minutes to 80 minutes. In addition, in order to obtain chitosan derivatives having different levels of alkyl carboxyl groups or free amine groups, the chitosan derivative powder having the aforementioned controlled deacetylation level may be added to 20 % NaOH at 5 w/v%, and then 1.0 to 1.5 w/v% of monochloroacetic acid may be added thereto and reacted.

In an embodiment, the chitosan derivative may have a degree of deacetylation of 70 % to 90 %. The degree of deacetylation indicates the level at which an acetyl group is removed from chitin, a precursor material of chitosan, and may indicate the % level of a unit in which R₁ is hydrogen or an alkylcarboxyl group in the Structural Formula 2. The degree of deacetylation of the chitosan derivative may be, for example, 70 % to 90 %, 75 % to 90 %, 80 % to 90 %, 85 % to 90 %, 70 % to 86 %, 75 % to 86 %, 80 % to 86 %, 85 % to 86 %, 70 % to 82 %, 75 % to 82 %, 80 % to 82 %, 70 % to 78 %, 75 % to 78 %, or 70 % to 74 %.

In an embodiment, the chitosan derivative may have a degree of substitution of an O-alkylcarboxyl group or an N-alkylcarboxyl group of 50 % to 90 %. The degree of substitution of an O-alkylcarboxyl group or an N-alkylcarboxyl group indicates the level of substitution of the hydroxyl group and amine group in the chitosan derivative with the -O-alkylcarboxyl group or the -N-alkylcarboxyl group, and in the Structural Formula 2, it may indicate the % level of the substituent in which the R₂ and R₃ are alkylcarboxyl groups. Here, the % level is a concept distinct from a conventional degree of substitution, which is described as a value calculated as the number of carboxymethyl groups per 100 anhydroglucosamine units and evaluated based on whether or not a carboxyl group is substituted. The degree of substitution of an O-alkylcarboxyl group or an N-alkylcarboxyl group of the chitosan derivative may be, for example, 50 % to 90 %, 55 % to 90 %, 60 % to 90 %, 65 % to 90 %, 70 % to 90 %, 75 % to 90 %, 80 % to 90 %, 85 % to 90 %, 50 % to 80 %, 55 % to 80 %, 60 % to 80 %, 65 % to 80 %, 70 % to 80 %, 75 % to 80 %, 50 % to 70 %, 55 % to 70 %, 60 % to 70 %, 65 % to 70 %, 50 % to 60 %, or 55 % to 60 %. At this time, if the degree of deacetylation and the degree of substitution of an O-alkylcarboxyl group or an N-alkylcarboxyl group of the chitosan derivative deviate from the above range, there is a problem in that stability under neutral conditions rapidly decreases and insoluble particles not dissolved in a solution increase.

In an embodiment, the chitosan derivative may have a level of free amine groups of 22 % to 90 %. The level of free amine groups represents a level of free amine groups (-NH₂) among the amine groups in the chitosan derivative, and may represent the % level of the substituent in which R₁ is hydrogen in the Structural Formula 2. The level of free amine groups of the chitosan derivative may be 22 % to 90 %, 25 % to 90 %, 30 % to 90 %, 35 % to 90 %, 40 % to 90 %, 45 % to 90 %, 50 % to 90 %, 55 % to 90 %, 60 % to 90 %, 65 % to 90 %, 70 % to 90 %, 75 % to 90 %, 80 % to 90 %, 85 % to 90 %, 22 % to 80 %, 25 % to 80 %, 30 % to 80 %, 35 % to 80 %, 40 % to 80 %, 45 % to 80 %, 50 % to 80 %, 55 % to 80 %, 60 % to 80 %, 65 % to 80 %, 70 % to 80 %, 75 % to 80 %, 22 % to 70 %, 25 % to 70 %, 30 % to 70 %, 35 % to 70 %, 40 % to 70 %, 45 % to 70 %, 50 % to 70 %, 55 % to 70 %, 60 % to 70 %, 65 % to 70 %, 22 % to 60 %, 25 % to 60 %, 30 % to 60 %, 35 % to 60 %, 40 % to 60 %, 45 % to 60 %, 50 % to 60 %, or 55 % to 60 %. At this time, if the level of free amine groups of the chitosan derivative deviates from the above range, there is a problem that gelation does not occur under *in vivo* conditions or the strength level of the formed hydrogel is very weak.

In an embodiment, the chitosan derivative may have a degree of deacetylation of 70 % to 90 %, a degree of substitution of an O-alkylcarboxyl group or an N-alkylcarboxyl group of 50 % to 90 %, and a level of free amine groups of 22 % to 90 %, and the chitosan derivative may, for example, have a degree of deacetylation of 70 % to 90 %, a degree of substitution of an O-alkylcarboxyl group or an N-alkylcarboxyl group of 50 % to 70 %, and a level of free amine groups of 24 % to 90 %.

According to an embodiment, it was confirmed that characteristics of the *in vivo* ion-sensitive hydrogel composition may be controlled according to a structure of the chitosan derivative. Specifically, a degree of deacetylation of the chitosan derivative and a level of terminal carboxyl groups (that is, a degree of substitution of an O-alkylcarboxyl group or an N-alkylcarboxyl group) may affect solubility and stability of the chitosan derivative under neutral conditions, and a level of free amine groups of the chitosan derivative may affect gelation under *in vivo* conditions and a viscoelastic level of a formed matrix. Accordingly, in the present embodiment, it was confirmed that functionality/effect as a biomaterial for tissue repair may be achieved by controlling the degree of deacetylation, the level of terminal carboxyl groups, and the level of free amine groups of chitosan derivatives, and based thereon, the present disclosure was completed.

The chitosan derivative may be contained at a concentration of 1 w/v% to 5 w/v% based on a total volume of the chitosan derivative solution, and a content of the chitosan derivative may be 1 w/v% to 5 w/v%, 1.5 w/v% to 5 w/v%, 2 w/v% to 5 w/v%, 2.5 w/v% to 5 w/v%, 3 w/v% to 5 w/v%, 3.5 w/v% to 5 w/v%, 4 w/v% to 5 w/v%, 4.5 w/v% to 5 w/v%, 1 w/v% to 4 w/v%, 1.5 w/v% to 4 w/v%, 2 w/v% to 4 w/v%, 2.5 w/v% to 4 w/v%, 3 w/v% to 4 w/v%, 3.5 w/v% to 4 w/v%, 1 w/v% to 3 w/v%, 1.5 w/v% to 3 w/v%, 2 w/v% to 3 w/v%, or 2.5 w/v% to 3 w/v% based on the total volume of the chitosan derivative solution.

If the content of the chitosan derivative is less than the above range, there is a problem in that gelation does not occur or a strength level of the formed hydrogel is very weak and the retention power is insufficient, and if the content of the chitosan derivative is greater than the above range, there is a problem that insoluble particles that are not dissolved in the solution increase.

In an embodiment, the composition for preparing the *in vivo* ion-sensitive hydrogel composition may further include an aqueous solution containing phosphate ions.

As used herein, the term "phosphate ions" refers to a component that contributes to forming a hydrogel under *in vivo* conditions by binding to amine groups of the chitosan derivative or to enhancing a strength of the formed hydrogel, and, as an example, may be provided in a form of an aqueous solution containing phosphate ions. The aqueous solution containing phosphate ions may include, for example, disodium hydrogen phosphate (Na₂HPO₄), monosodium hydrogen phosphate monobasic (NaH₂PO₄), diammonium hydrogen phosphate ((NH₄)₂HPO₄), ammonium dihydrogen phosphate (NH₄H₂PO₄), sodium phosphate tribasic (Na₃PO₄), potassium phosphate dibasic (K₂HPO₄), potassium phosphate monobasic (KH₂PO₄), dimethyl phosphate (C₂H₇PO₄), monomagnesium phosphate (Mg(H₂PO₄)₂), magnesium phosphate dibasic (MgHPO₄), lithium dihydrogen phosphate (LiH₂PO₄), lithium phosphate (LiPO₄), calcium hydrogen orthophosphate hydrate (CaHPO₄· 2H₂O), or calcium hydrogen orthophosphate (CaHPO₄), but, as long as it is a material capable of providing a phosphate group or a phosphate that may bind to amine groups of the chitosan derivative, it may be extended without limitation.

The composition may contain an aqueous solution containing phosphate ions in an amount of 7 to 15 wt% based on the total weight of the composition, and for example, the content of the aqueous solution containing phosphate ions may be 7 to 9 wt%, 7 to 11 wt%, 7 to 13 wt%, 9 to 11 wt%, 9 to 13 wt%, 9 to 15 wt%, 11 to 13 wt%, 11 to 15 wt%, or 13 to 15 wt%.

In an embodiment, the composition for preparing the *in vivo* ion-sensitive hydrogel composition may further include a liquid formulation containing glycerol.

In an embodiment, the liquid formulation may be isolated in a separate space within the container from the aforementioned solution containing the chitosan derivative and/or the aqueous solution containing phosphate ions. Therefore, the solution containing chitosan and phosphate ions and the liquid preparation containing glycerol may be maintained and stored in liquid form, respectively, under conditions prior to use, and, if necessary, may also be stored in a frozen state for long-term storage.

The liquid formulation containing glycerol may be mixed with a solution containing the chitosan derivative and an aqueous solution containing phosphate ions to adjust the level of covalent bonding within the mixture, thereby imparting viscoelastic properties to a biomaterial for tissue repair.

The glycerol may be contained at a concentration of 0.01 v/v% to 1 v/v% based on the total volume of the liquid preparation or composition, and the content of the glycerol may be 0.01 v/v% to 1 v/v%, 0.05 v/v% to 1 v/v%, 0.1 v/v% to 1 v/v%, 0.5 v/v% to 1 v/v%, 0.01 v/v% to 0.5 v/v%, 0.05 v/v% to 0.5 v/v%, 0.1 v/v% to 0.5 v/v%, 0.01 v/v% to 0.1 v/v%, 0.05 v/v% to 0.1 v/v%, or 0.01 v/v% to 0.05 v/v% based on the total volume of the liquid preparation or composition.

Another aspect is to provide a method of preparing an *in vivo* ion-sensitive hydrogel composition, the method including mixing a solution containing the chitosan derivative satisfying the following conditions and an aqueous solution containing phosphate ions:
(a) having a degree of deacetylation of 70 % or more;
(b) 50 % or more of the hydroxyl groups and amine groups in the chitosan derivative are substituted with O-alkylcarboxyl groups or N-alkylcarboxyl groups; and
(c) 22 % or more of the total amine groups in the chitosan derivative shall be free amine groups (-NH₂).

Another aspect is to provide an *in vivo* ion-sensitive hydrogel composition prepared by the method.

Another aspect is to provide a method of administering an *in vivo* ion-sensitive hydrogel composition, the method including mixing the aforementioned solution containing the chitosan derivative and the aqueous solution containing phosphate ions, and injecting the mixed liquid formulation into the intradermis of a subject.

The method of preparing the *in vivo* ion-sensitive hydrogel composition, the *in vivo* ion-sensitive hydrogel composition prepared by the method, or the method of administering the *in vivo* ion-sensitive hydrogel composition includes or uses the composition for preparing the aforementioned *in vivo* ion-sensitive hydrogel composition, and therefore, overlapping descriptions therebetween are omitted.

According to an aspect, the *in vivo* ion-sensitive hydrogel composition or tissue repair biomaterial manufactured by the method maintains high solubility of chitosan derivatives and stability of the material under neutral conditions, unlike existing tissue repair biomaterials that are dissolved and used under acidic conditions, thereby alleviating pain during *in vivo* administration and facilitating mixing with other materials or components. In addition, the *in vivo* ion-sensitive hydrogel composition or biomaterial for tissue repair has both the characteristics of a liquid preparation that may be injected at room temperature and the ability to gel under *in vivo* conditions to form a viscoelastic matrix that matches the shape and characteristics of various tissues, and therefore, may be widely used in various fields.

### Advantageous Effects of Invention

The composition according to an aspect includes a solution containing a chitosan derivative having a controlled degree of deacetylation, a degree of substitution of alkyl carboxyl groups, and a level of free amine groups, and through this, unlike existing biomaterials for tissue repair, high solubility and stability under neutral conditions may be achieved.

The composition according to an aspect is not only stable as a material, but may also be gelled under *in vivo* conditions to form a matrix with various viscoelasticities, thereby providing a biomaterial for tissue repair that matches the shape and characteristics of various tissues.

### Brief Description of Drawings

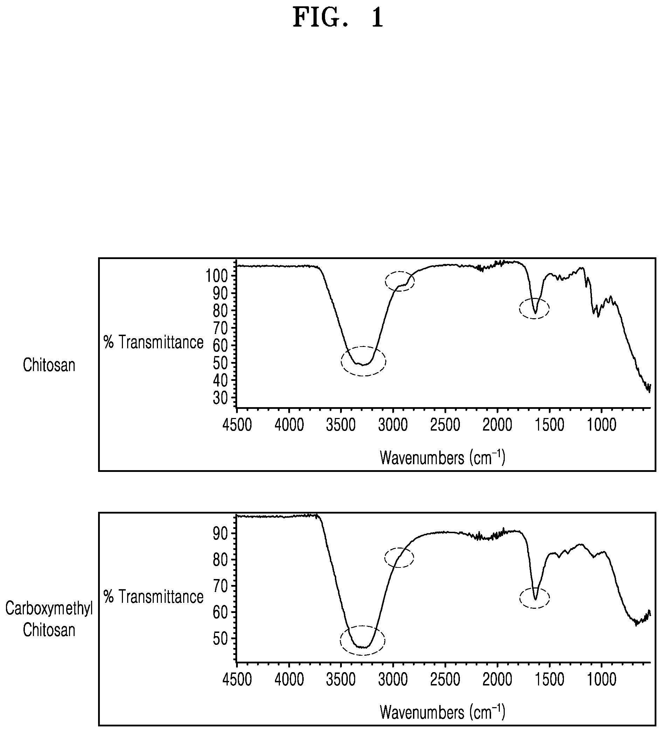

FIG. 1 shows the results of determining substitution of methylcarboxyl groups in a chitosan derivative by FT-IR spectroscopy, in a solution including the chitosan derivative according to an embodiment.

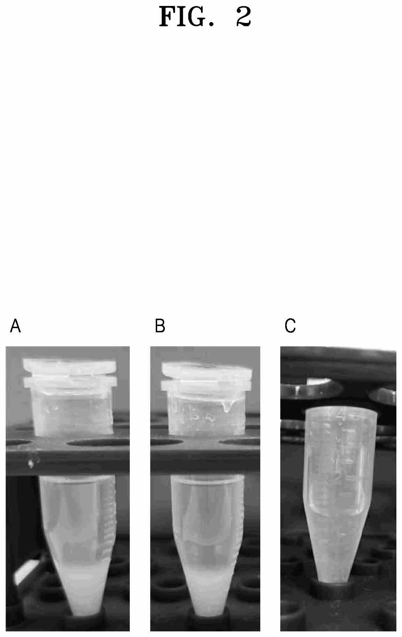

FIG. 2 shows the results of determining the change in solubility of a chitosan derivative in a neutral solvent according to the degree of substitution of methylcarboxyl groups, in a solution including a chitosan derivative having a degree of deacetylation of 50 %, according to an embodiment, wherein A of FIG. 2 shows the results of visually checking a chitosan derivative solution having a degree of substitution of methylcarboxyl groups of 0 %, B of FIG. 2 shows the results of visually checking a chitosan derivative solution having a degree of substitution of methylcarboxyl groups of 50 %, and C of FIG. 2 shows the results of visually checking a chitosan derivative solution having a degree of substitution of methylcarboxyl groups of 70 %.

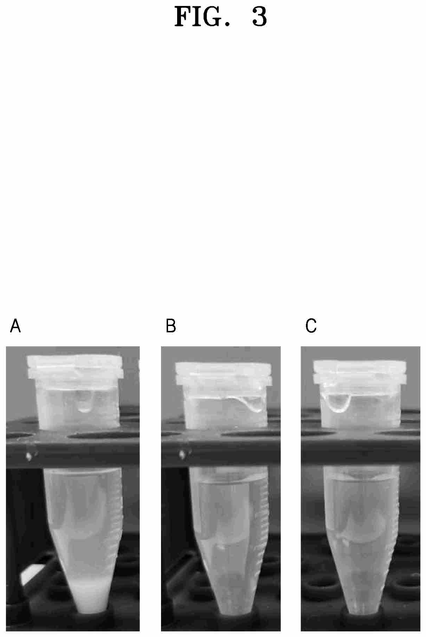

FIG. 3 shows the results of checking the change in solubility of a chitosan derivative in a neutral solvent according to the degree of substitution of methylcarboxyl groups in a solution including the chitosan derivative having a degree of deacetylation of 70 %, according to an embodiment, wherein A of FIG. 3 shows the results of visually checking a chitosan derivative solution having a degree of substitution of methylcarboxyl groups of 0 %, B of FIG. 3 shows the results of visually checking a chitosan derivative solution having a degree of substitution of methylcarboxyl groups of 50 %, and C of FIG. 3 shows the results of visually checking a chitosan derivative solution having a degree of substitution of methylcarboxyl groups of 70 %.

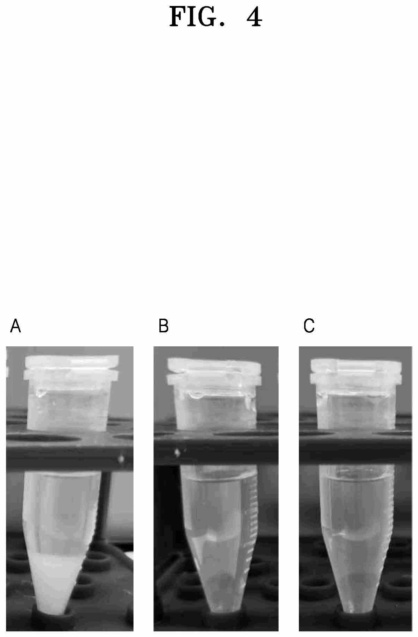

FIG. 4 shows the results of checking a change in solubility of a chitosan derivative in a neutral solvent according to the degree of substitution of methylcarboxyl groups in a solution including the chitosan derivative having a degree of deacetylation of 90 %, according to an embodiment, wherein A of FIG. 4 shows the results of visually checking a chitosan derivative solution having a degree of substitution of methylcarboxyl groups of 0 %, B of FIG. 4 shows the results of visually checking a chitosan derivative solution having a degree of substitution of methylcarboxyl groups of 50 %, and C of FIG. 4 shows the results of visually checking a chitosan derivative solution having a degree of substitution of methylcarboxyl groups of 70 %.

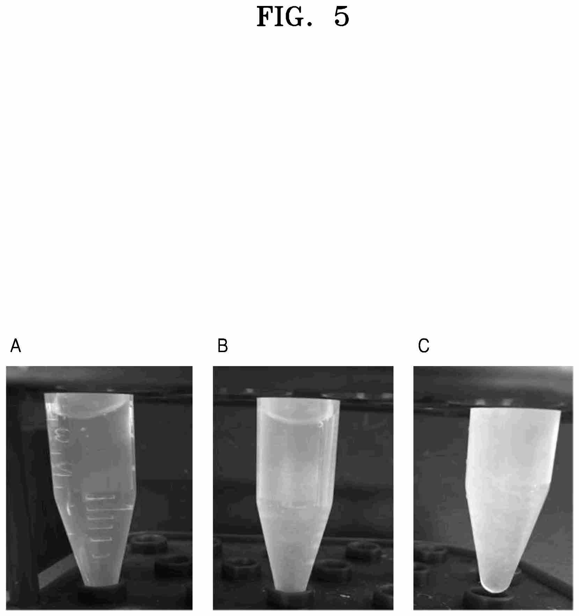

FIG. 5 shows the results of checking the change in solubility of a chitosan derivative according to the change in pH of a solution, in a solution including the chitosan derivative having a degree of deacetylation of 90 % and a degree of substitution of methylcarboxyl groups of 70 %, according to an embodiment, wherein A of FIG. 5 shows the results of visually checking a chitosan derivative solution at pH 6.8, B of FIG. 5 shows the results of visually checking a chitosan derivative solution at pH 6.4, and C of FIG. 5C shows the results of visually checking a chitosan derivative solution at pH 6.1.

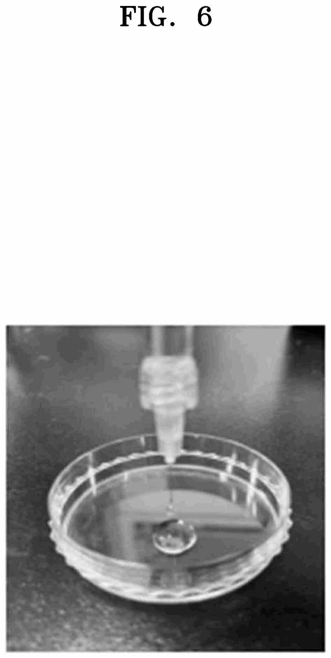

FIG. 6 shows the results of visually checking characteristics of an *in vivo* ion-sensitive hydrogel composition as an injectable liquid formulation, according to an embodiment.

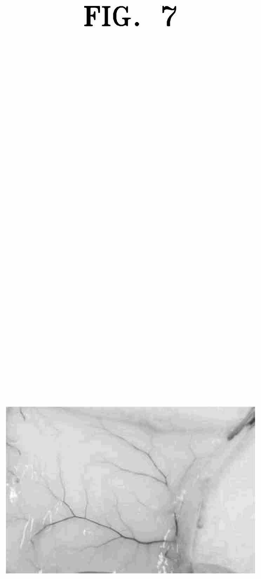

FIG. 7 shows the results of checking, using an animal model, whether gelation occurs *in vivo* in an *in vivo* ion-sensitive hydrogel composition including a chitosan derivative having a level of free amine groups of 21 %, according to an embodiment.

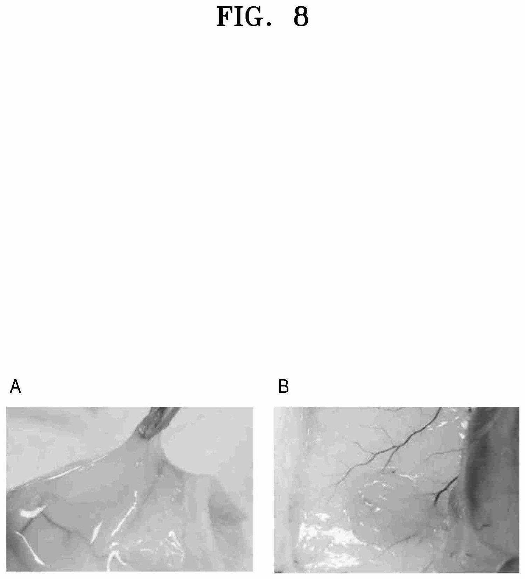

FIG. 8 shows the results of checking, using an animal model, whether gelation occurs *in vivo* in an *in vivo* ion-sensitive hydrogel composition including a chitosan derivative having a level of free amine groups of 22 % or more, according to an embodiment, wherein A of FIG. 8 is a result checking whether gelation occurs in an *in vivo* ion-sensitive hydrogel composition including a chitosan derivative having a level of free amine groups of 34 %, and B of FIG. 8 is a result checking whether gelation occurs in an *in vivo* ion-sensitive hydrogel composition including a chitosan derivative having a level of free amine groups of 90 %.

### Mode for the Invention

Hereinafter, preferred examples are presented to aid in the understanding of the present disclosure. However, the following examples are provided only to aid understanding of the present disclosure, and the present disclosure is not limited by the following examples.

### [Example]

### Example 1. Evaluation of Dissolution Level of Chitosan Derivative Solutions Under Neutral Conditions

Existing biomaterials for tissue repair including chitosan exhibit a characteristic of being dissolved in an acidic solution, and thus cause pain during *in vivo* administration, and when chitosan precipitates under neutral conditions or is mixed with another material or component, there exists a problem in that stability of the material is drastically reduced. Accordingly, in the present example, a chitosan derivative solution having high solubility under neutral conditions was to be prepared.

### 1-1. Preparation of Chitosan Derivative Solution for Preparation of in Vivo Ion-sensitive Hydrogel Composition

In the present example, solutions containing chitosan derivatives having different degrees of deacetylation (%) and degrees of substitution of a methylcarboxyl group (%) were prepared as follows. As shown in Table 1 below, the chitosan derivatives were first classified into three groups based on degrees of deacetylation (50 %, 70 %, 90 %), and each of the classified groups was reclassified into three experimental groups based on degrees of substitution of a methylcarboxyl group in the chitosan derivatives, thereby establishing a total of nine experimental groups. Here, the degree of deacetylation (%) indicates a level at which an acetyl group is removed from chitin, which is a precursor material of the chitosan derivative, and the degree of substitution of a methylcarboxyl group (%) indicates a level at which hydroxyl groups and amine groups in the chitosan derivative are substituted with -O-methylcarboxyl groups or -N-methylcarboxyl groups. In addition, PBS (pH 7.4), which is a neutral solvent, was used as a solvent in the chitosan derivative solution.

**[Table 1]**

| **Classification** | **Degree of deacetylation (%)** | **Degree of methylcarboxy substitution (%)** | **Experimental group** |
|---|---|---|---|
| G1 | 50 | 0 | G1-1 |
| | | 50 | G1-2 |
| | | 70 | G1-3 |
| G2 | 70 | 0 | G2-1 |
| | | 50 | G2-2 |
| | | 70 | G2-3 |
| G3 | 90 | 0 | G3-1 |
| | | 50 | G3-2 |
| | | 70 | G3-3 |

### (1) Preparation of Experimental Group G1-1

Chitin 10 g was reacted in 100 mL of a 50 % NaOH solution at 100 °C for 30 minutes. Immediately after completion of the reaction, washing was performed by adding water. The deacetylated chitosan was finally dried at 80 °C to obtain a chitosan powder having a degree of deacetylation of 50 %.

### (2) Preparation of Experimental Group G1-2

Chitin 10 g was reacted in 100 mL of a 50 % NaOH solution at 100 °C for 30 minutes. Immediately after completion of the reaction, washing was performed by adding water. The deacetylated chitosan was finally obtained by drying at 80 °C. Thereafter, the chitosan powder having the degree of deacetylation of 50 % was added to 20 % NaOH at 5 w/v%, monochloroacetic acid (1.0 w/v%) was added thereto, and was reacted at 40 °C. Thereafter, neutralization was performed with 10 % acetic acid, followed by purification with methanol, to finally obtain a chitosan derivative having a degree of deacetylation of 50 % and a degree of substitution of a methylcarboxyl group of 50 %.

### (3) Preparation of Experimental Group G1-3

Chitin 10 g was reacted in 100 mL of a 50 % NaOH solution at 100 °C for 30 minutes. Immediately after completion of the reaction, washing was performed by adding water. The deacetylated chitosan was finally obtained by drying at 80 °C. Thereafter, the chitosan powder having the degree of deacetylation of 50 % was added to 20 % NaOH at 5 w/v%, monochloroacetic acid (1.5 w/v%) was added thereto, and was reacted at 40 °C. Thereafter, neutralization was performed with 10 % acetic acid, followed by purification with methanol, to finally obtain a chitosan derivative having a degree of deacetylation of 50 % and a degree of substitution of a methylcarboxyl group of 70 %.

### (4) Preparation of Experimental Group G2-1

Chitin 10 g was reacted in 100 mL of a 50 % NaOH solution at 100 °C for 60 minutes. Immediately after completion of the reaction, washing was performed by adding water. The deacetylated chitosan was finally dried at 80 °C to obtain a chitosan powder having a degree of deacetylation of 70 %.

### (5) Preparation of Experimental Group G2-2

Chitin 10 g was reacted in 100 mL of a 50 % NaOH solution at 100 °C for 60 minutes. Immediately after completion of the reaction, washing was performed by adding water. The deacetylated chitosan was finally obtained by drying at 80 °C. Thereafter, the chitosan powder having the degree of deacetylation of 70 % was added to 20 % NaOH at 5 w/v%, monochloroacetic acid (1.0 w/v%) was added thereto, and was reacted at 40 °C. Thereafter, neutralization was performed with 10 % acetic acid, followed by purification with methanol, to finally obtain a chitosan derivative having a degree of deacetylation of 70 % and a degree of substitution of a methylcarboxyl group of 50 %.

### (6) Preparation of Experimental Group G2-3

Chitin 10 g was reacted in 100 mL of a 50 % NaOH solution at 100 °C for 60 minutes. Immediately after completion of the reaction, washing was performed by adding water. The deacetylated chitosan was finally obtained by drying at 80 °C. Thereafter, the chitosan powder having the degree of deacetylation of 70 % was added to 20 % NaOH at 5 w/v%, monochloroacetic acid (1.5 w/v%) was added thereto, and was reacted at 40 °C. Thereafter, neutralization was performed with 10 % acetic acid, followed by purification with methanol, to finally obtain a chitosan derivative having a degree of deacetylation of 70 % and a degree of substitution of a methylcarboxyl group of 70 %.

### (7) Preparation of Experimental Group G3-1

Chitin 10 g was reacted in 100 mL of a 50 % NaOH solution at 100 °C for 150 minutes. Immediately after completion of the reaction, washing was performed by adding water. The deacetylated chitosan was finally dried at 80 °C to obtain a chitosan powder having a degree of deacetylation of 90 %.

### (8) Preparation of Experimental Group G3-2

Chitin 10 g was reacted in 100 mL of a 50 % NaOH solution at 100 °C for 150 minutes. Immediately after completion of the reaction, washing was performed by adding water. The deacetylated chitosan was finally obtained by drying at 80 °C. Thereafter, the chitosan powder having the degree of deacetylation of 90 % was added to 20 % NaOH at 5 w/v%, monochloroacetic acid (1.0 w/v%) was added thereto, and was reacted at 40 °C. Thereafter, neutralization was performed with 10 % acetic acid, followed by purification with methanol, to finally obtain a chitosan derivative having a degree of deacetylation of 90 % and a degree of substitution of a methylcarboxyl group of 50 %.

### (9) Preparation of Experimental Group G3-3

Chitin 10 g was reacted in 100 mL of a 50 % NaOH solution at 100 °C for 150 minutes. Immediately after completion of the reaction, washing was performed by adding water. The deacetylated chitosan was finally obtained by drying at 80 °C. Thereafter, the chitosan powder having the degree of deacetylation of 90 % was added to 20 % NaOH at 5 w/v%, monochloroacetic acid (1.5 w/v%) was added thereto, and was reacted at 40 °C. Thereafter, neutralization was performed with 10 % acetic acid, followed by purification with methanol, to finally obtain a chitosan derivative having a degree of deacetylation of 90 % and a degree of substitution of a methylcarboxyl group of 70 %.

Thereafter, with respect to the chitosan derivatives having different degrees of deacetylation and degrees of substitution of a methylcarboxyl group obtained as described above, substitution of a methylcarboxyl group and a level of substitution in the chitosan derivatives were confirmed by measuring intensity of a band through FT-IR spectroscopy (see FIG. 1) and by measuring content of free amines using a Ninhydrin assay.

### 1-2. Evaluation of Dissolution Level of Chitosan Derivative Under Neutral Solvent Conditions

In the present example, a dissolution level of the chitosan derivative solution of Example 1-1 was evaluated to confirm an effect of a degree of deacetylation of the chitosan derivative and/or a degree of substitution of a methylcarboxyl group on a dissolution level of the chitosan derivative with respect to a neutral solvent. To this end, a dissolution level of the chitosan derivative solutions of Example 1-1, having different degrees of deacetylation and degrees of substitution of a methylcarboxyl group, was visually evaluated under room temperature conditions.

As a result, as shown in FIG. 2, all G1 groups including a chitosan derivative having a degree of deacetylation of 50 % were observed to generate precipitates with respect to the neutral solvent or to be in a turbid state due to a low dissolution level of the chitosan derivative. On the other hand, as shown in FIG. 3 and FIG. 4, G2 groups or G3 groups including a chitosan derivative having a degree of deacetylation of 70 % or 90 % were observed as transparent solutions due to an increased dissolution level of the chitosan derivative with respect to the neutral solvent. In particular, such an effect was confirmed from G2-2 and G3-2 groups having a degree of substitution of a methylcarboxyl group of 50 % or more, and was more clearly observed as the degree of substitution of the methylcarboxyl group increased.

### Example 2. Evaluation of Dissolution Level of Chitosan Derivative According to pH Change

In the present example, a change in a dissolution level of the chitosan derivative solution of Example 1-1 according to pH change was evaluated to verify a difference from existing biomaterials for tissue repair having a characteristic of being dissolved under acidic conditions. To this end, in the same manner as in Example 1, a solution including, at 2 w/v%, a chitosan derivative having a degree of deacetylation of 90 % and a degree of substitution of a methylcarboxyl group of 70 % was prepared. However, in the chitosan derivative solution, 0.01 N HCl aqueous solution, 0.015 N HCl aqueous solution, and 0.02 N HCl aqueous solution were used as solvents to adjust pH levels of the solutions to 6.8, 6.4, and 6.1, respectively. Thereafter, in the same manner as in Example 1-2, a dissolution level of chitosan derivative solutions having different pH levels was visually evaluated at room temperature.

As a result, as shown in FIG. 5, whereas the solution was observed as a transparent solution under neutral conditions due to a high dissolution level of the chitosan derivative, the solutions were observed to generate precipitates or to be in a turbid state under weakly acidic conditions of pH 6.5 or less due to a low dissolution level of the chitosan derivative.

Taken together, the experimental results indicate that, by controlling a degree of deacetylation (70 % or more) and a degree of substitution of a methylcarboxyl group (50 % or more) of the chitosan derivative, it is possible to prepare a chitosan derivative solution or a biomaterial including the same, having a high solubility and stability under neutral conditions, unlike existing biomaterials for tissue repair.

### Example 3. Evaluation of in Vivo Ion-sensitive Level According to Level of Free Amine Groups

To be used as a biomaterial for tissue repair, the material must exist in an injectable liquid form under room temperature conditions, and after *in vivo* administration, that is, *in vivo*, gelation must proceed to form an effective volume. To this end, in the present example, an effect of a free amine group in the chitosan derivative on *in vivo* ion-sensitive behavior was confirmed.

### 3-1. Preparation of an in Vivo Ion-sensitive Hydrogel Composition

In the present example, a solution including a chitosan derivative having different levels (%) of free amine groups were prepared as follows.

**[Table 2]**

| **Experimental group** | **Degree of deacetylation (%)** | **Degree of methylcarboxy substitution (%)** | **Free amine (%)** |
|---|---|---|---|
| N1 | 90 | 50 | 21 |
| N2 | | 70 | 34 |
| N3 | | 0 | 90 |

As shown in Table 2, the degrees of deacetylation (%) and the degrees of substitution of a methylcarboxyl group (%) are as shown in Table 1, and the free amine group (%) represent levels of free amine groups (-NH₂) among amine groups in the chitosan derivative, and a neutral solvent of disodium phosphate solution was used as a solvent in the chitosan derivative solution. In the present example, the experimental groups (N1, N2, N3) were prepared by performing N,O-random carboxymethyl substitution, and, in the same manner as in Example 1-1, levels of free amine groups were adjusted by adjusting a degree of substitution of a methylcarboxyl group.

Thereafter, by mixing the 2 w/v% chitosan derivative solution and 2 v/v% disodium hydrogen phosphate (Na₂HPO₄), an *in vivo* ion-sensitive hydrogel composition in a liquid form injectable under room temperature conditions was prepared, as shown in FIG. 6.

### 3-2. Evaluation of in Vivo Ion-sensitiveness Using Animal Models

In the present example, the gelation of the *in vivo* ion-sensitive hydrogel composition of Example 3-2 was evaluated using an animal model, thereby checking the effect of the level of free amine groups of chitosan derivatives on the *in vivo* ion-sensitive behavior. For this purpose, 0.5 ml of the *in vivo* ion-sensitive hydrogel composition of Example 3-1 was injected intradermally into a hairless mouse. One day after administration of the hydrogel composition, matrix formation due to gelation progression was visually confirmed, and their storage modulus (G') was measured.

As a result, as shown in FIG. 7, N1 groups including a chitosan derivative having a level of free amine group of 21 % did not form a matrix *in vivo*. On the other hand, as shown in FIG. 8, N2 groups including a chitosan derivative having a level of free amine group of 34 % formed a matrix having a storage elastic modulus of 31.97±1.58 Pa, which is similar to that of a hyaluronic acid filler having a low degree of cross-linking, and N3 groups including a chitosan derivative having a level of free amine group of 90 % were confirmed to form a matrix having a storage elastic modulus of 154.31±13.21 Pa. By controlling the elastic modulus of the matrix in the manner presented herein, the *in vivo* ion-sensitive hydrogel composition forming a matrix having a storage elastic modulus of 5 to 50 Pa may be applied to medical materials such as joint injectable formulations and skin boosters, etc. and the *in vivo* ion-sensitive hydrogel composition forming a matrix having a storage elastic modulus of 100 Pa or more may be applied to medical materials for subcutaneous repair.

Taken together, the experimental results indicate that, by controlling a degree of deacetylation and a degree of substitution of a methylcarboxyl group of the chitosan derivative, together with a level of free amine groups of the chitosan derivative, it is possible not only to solve problems of existing tissue repairing biomaterials, but also to prepare a biomaterial having both characteristics of being an injectable liquid formulation under room temperature conditions and forming a matrix having viscoelasticity corresponding to shapes and characteristics of various tissues due to gelation under *in vivo* conditions.

The foregoing description of the present disclosure is for illustrative purposes only, and one that has ordinary skill in the art to which the present disclosure belongs will understand that the present disclosure may be readily adapted to other specific forms without altering the technical ideas or essential features of the present disclosure. Therefore, the examples described above should be understood in all respects as illustrative and not restrictive.

## Claims

1. A composition for preparing an *in vivo* ion-sensitive hydrogel composition comprising a solution containing a chitosan derivative satisfying the following conditions:
(a) having a degree of deacetylation of 70 % or more;
(b) 50 % or more of hydroxyl groups and amine groups in the chitosan derivative are substituted with O-alkylcarboxyl groups or N-alkylcarboxyl groups; and
(c) 22 % or more of total amine groups in the chitosan derivative are free amine groups (-NH₂).

2. The composition of claim 1, wherein the chitosan derivative has a degree of deacetylation of 70 % to 90 %.

3. The composition of claim 1, wherein 50 % to 90 % of hydroxyl groups and amine groups in the chitosan derivative are substituted with an O-methylcarboxyl group or an N-methylcarboxyl group.

4. The composition of claim 1, wherein 22 % to 90 % of total amine groups in the chitosan derivative are free amine groups.

5. The composition of claim 1, wherein an average molecular weight of the chitosan derivative is 50,000 to 3,000,000 Da.

6. The composition of claim 1, wherein the solution has a pH of pH 6.5 to pH 7.5.

7. The composition of claim 1, wherein the chitosan derivative is contained at a concentration of 2 to 5 w/v% based on the total volume of the solution.

8. The composition of claim 1, wherein the composition further comprises an aqueous solution containing phosphate ions.

9. The composition of claim 8, wherein the aqueous solution containing phosphate ions comprises at least one phosphate selected from the group consisting of disodium hydrogen phosphate (Na₂HPO₄), monosodium hydrogen phosphate monobasic (NaH₂PO₄), diammonium hydrogen phosphate ((NH₄)₂HPO₄), ammonium dihydrogen phosphate (NH₄H₂PO₄), sodium phosphate tribasic (Na₃PO₄), potassium phosphate dibasic (K₂HPO₄), potassium phosphate monobasic (KH₂PO₄), dimethyl phosphate (C₂H₇PO₄), monomagnesium phosphate (Mg(H₂PO₄)₂), magnesium phosphate dibasic (MgHPO₄), lithium dihydrogen phosphate (LiH₂PO₄), lithium phosphate (LiPO₄), calcium hydrogen orthophosphate hydrate (CaHPO₄·2H₂O), and calcium hydrogen orthophosphate (CaHPO₄).

10. The composition of claim 1, wherein the composition further comprises a liquid preparation containing glycerol.

11. The composition of claim 1, wherein the composition is a transparent liquid formulation under neutral conditions and forms a gel formulation *in vivo*.

12. A method of preparing an *in vivo* ion-sensitive hydrogel composition, the method comprising mixing a solution containing a chitosan derivative satisfying the following conditions and an aqueous solution containing phosphate ions:
(a) having a degree of deacetylation of 70 % or more;
(b) 50 % or more of hydroxyl groups and amine groups in the chitosan derivative are substituted with O-alkylcarboxyl groups or N-alkylcarboxyl groups; and
(c) 22 % or more of total amine groups in the chitosan derivative are free amine groups (-NH₂).

13. The method of claim 12, wherein 50 % to 90 % of hydroxyl groups and amine groups in the chitosan derivative are substituted with an O-methylcarboxyl group or an N-methylcarboxyl group.

14. The method of claim 12, wherein the solution containing the chitosan derivative has a pH of pH 6.5 to pH 7.5.

15. The method of claim 12, wherein the aqueous solution containing phosphate ions comprises at least one phosphate selected from the group consisting of disodium hydrogen phosphate, monosodium hydrogen phosphate monobasic, diammonium hydrogen phosphate, ammonium dihydrogen phosphate, sodium phosphate tribasic, potassium phosphate dibasic, potassium phosphate monobasic, dimethyl phosphate, monomagnesium phosphate, magnesium phosphate dibasic, lithium dihydrogen phosphate, lithium phosphate, calcium hydrogen orthophosphate hydrate, and calcium hydrogen orthophosphate.

16. The method of claim 12, further comprising, after the mixing, adding and mixing a liquid preparation containing glycerol.
